(19) Europäisches Patentamt / European Patent Office / Office européen des brevets

(11) EP 4 286 373 A1

(12) EUROPEAN PATENT APPLICATION

(43) Date of publication:
**06.12.2023 Bulletin 2023/49**

(21) Application number: **22176144.8**

(22) Date of filing: **30.05.2022**

(51) International Patent Classification (IPC):
***C07D 249/04*** *(2006.01)* ***A61P 31/04*** *(2006.01)*

(52) Cooperative Patent Classification (CPC):
**A61P 31/04; C07D 249/04**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Technische Universität Berlin**
**10623 Berlin (DE)**

(72) Inventors:
- **SÜSSMUTH, Roderich**
  **10629 Berlin (DE)**
- **WESTON, John**
  **65779 Kelkheim (DE)**
- **KLEEBAUER, Leonardo**
  **10623 Berlin (DE)**
- **ZBOROVSKY, Lieby**
  **14052 Berlin (DE)**
- **HOMMERNICK, Kay**
  **10551 Berlin (DE)**

(74) Representative: **Maikowski & Ninnemann Patentanwälte Partnerschaft mbB**
**Postfach 15 09 20**
**10671 Berlin (DE)**

# (54) NOVEL ALBICIDIN DERIVATIVES, THEIR USE AND SYNTHESIS

(57) The present invention relates to a chemical compound according to general formula (1)

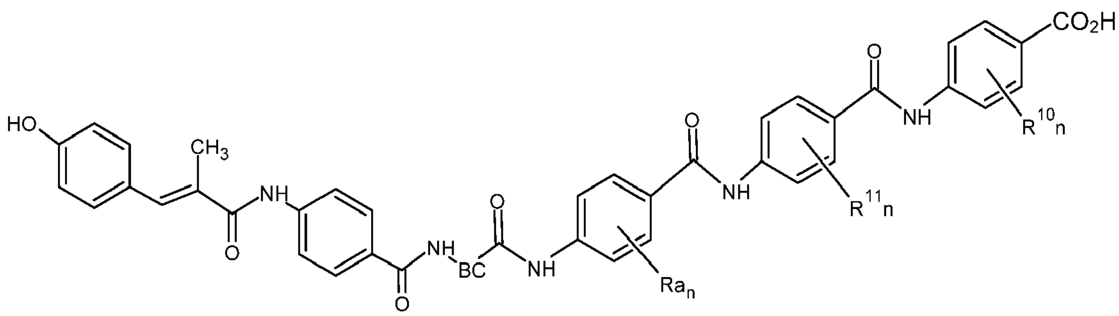

EP 4 286 373 A1

## Description

The present invention relates to albicidin derivatives.

Albicidin is a natural product, isolated from *Xanthomonas albilineans* and heterologously expressed in *Xanthomonas axonopodis* pv *vesicatoria*. Its structure (see below) is based on peptides and amino acids, but it does not contain any proteinogenic amino acids.

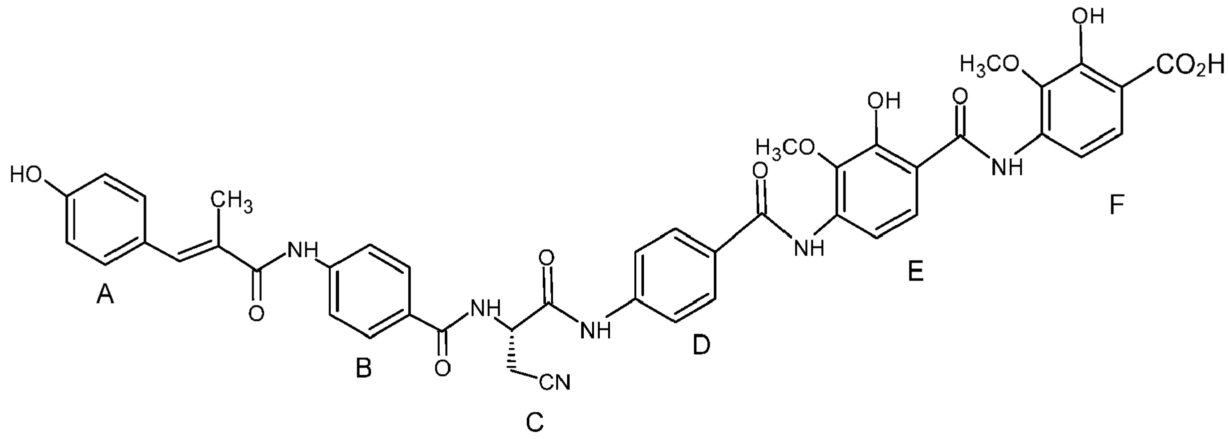

Albicidin is, on the one hand, a causative agent of the leaf scald disease in sugar cane and on the other hand a DNA-gyrase-inhibitor of prokaryotic cells (gram-positive and -negative). The mentioned properties make the natural product albicidin a potential antibiotic.

The known molecular structure of albicidin and available synthetic routes allows the development of a plurality of novel derivatives that may exhibit potential antimicrobial activities.

It is known that peptides as drugs can be hydrolysed relatively quickly by enzymes in the body, i.e. they are degraded. To counteract, some groups have been introduced that are supposed to reduce the rate of hydrolysis by increasing steric hindrance, to give compounds which are more stable in the presence of enzymes.

Many of the groups introduced but not all, which increase steric hindrance, are electron withdrawing in nature.

Some bacteria have already developed resistance to the natural product albicidin, and to counteract this, various chemical modifications have been made to the molecule. Specifically, the bacterium Pantoea dispersa is resistant to albicidin. An enzyme of the pathogen, more precisely the serine endopeptidase, is able to cleave albicidin between the building block D and E. The individual fragments have no antibacterial effect. Thus, it is desirable that the cleavage of the peptide bond should be prevented.

The bacterium *Pantoea dispersa* is a rare gram-negative microorganism that is mainly pathogenic to plants, but there are also known cases where humans have been infected with the pathogen. Treatment with the usual antibiotics: Clavulanic acid, piperacillin/tazobactam, all cephalosporins, all carbapenems, all aminoglycosides, fluoroquinolones and other antibiotics is ineffective. Only treatment with tigecycline (MIC = 1) or colistin (MIC $\leq$ 0.5) has been successful up to now. The consequences of no treatment or an ineffective treatment are progressive, systematic inflammatory reactions and even death.

Many of the groups introduced but not all, which increase steric hindrance and thus reduce the rate of hydrolysis, are rather lipophilic. Introducing lipophilic groups can be expected to increase the proportion of active substance bound to human serum, consequently the proportion of "free" active substance decreases and the concentration-dependent activity decreases. In this case it has been found that introducing such groups has little effect on the activity as measured. Accordingly, the antibacterial activity value (MIC) in the presence of human serum remains the same even when lipophilic substituents are introduced..

The problem underlying the present invention is therefore provision of new compounds, which comprise antibiotic properties, solubility and bioavailability, method of their synthesis and their use. This problem is attained by the subject-matter of the independent claims.

## Terms and Definitions

The term "purity" as used in the context of the present specification with respect to a preparation of a certain compound refers to the content of said compound relative to the sum of all compounds contained in the preparation. The term "compound" in this context is to be understood as a compound according to the invention (or any specific embodiments thereof) as well as any salts, hydrates or solvates thereof. Thus, the respective salts, hydrates or solvates

are not considered as impurities according to the previous definition. The "purity" of a compound may be determined using elemental analysis, HPLC analysis using UV diode array detection also in combination with mass spectrometry detection, or quantitative NMR analysis.

The term "substituted" refers to the addition of a substituent group to a parent moiety. "Substituent groups" can be protected or unprotected and can be added to one available site or to many available sites in a parent moiety.

**Description of the invention**

According to one aspect, the invention relates to compounds having a molecular structure as defined by formula (1)

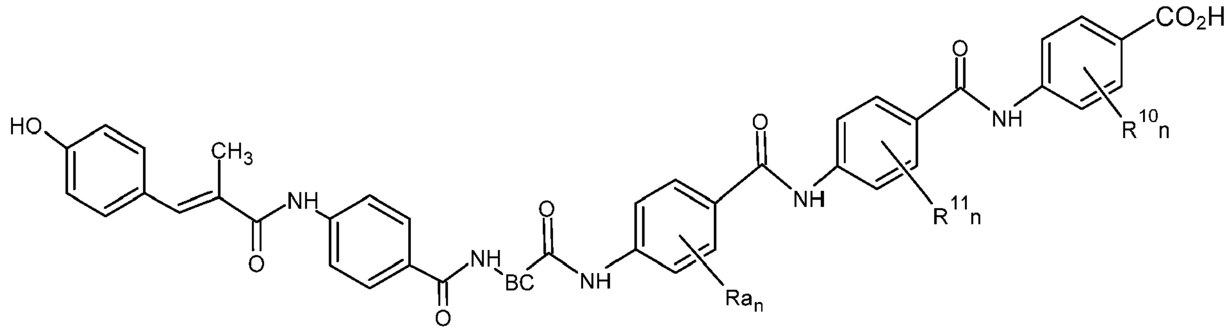

a) with BC being

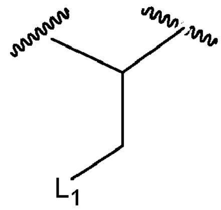

with $L_1$ being a substituted or unsubstituted C5-C6 aromatic heterocycle,

b) with n of $R^{10}_n$ and n of $R^{11}_n$ being independently from each other 0, 1, 2, 3 or 4, in particular n of $R^{10}_n$ and n of $R^{11}_n$ being 0, 1, 2 or 3, more particular n of $R^{10}_n$ and n of $R^{11}_n$ being 1 and 2;
with each $R^{10}$ and $R^{11}$ being selected independently from any other $R^{10}$ and $R^{11}$ from -OH, -F, -Cl, -Br, -I, -CCH, -CN, -$OC_1$-$C_6$ alkyl, in particular from -OH, -F, -$OCH_3$,-$OC_2H_5$, -$OiC_3H_7$, -$OnC_3H_7$,-$OCF_{3,}$; and

c) with n of $R^a_n$ being 0, 1, 2, 3 or 4, in particular 0, 1, 2 or 3, more particular being 1 and 2;
with $R_a$ being a substituent selected from halogen, in particular-Cl, - F, - Br, -I,-$NO_2$, -$NH_2$, -$CO_2H$, -CN, - $CF_3$,

In one embodiment, $R_a$ being an electron withdrawing moiety is selected from -Cl, - F, - Br, -I, - $CF_3$.

In one embodiment, the moiety $L_1$ is a five membered aromatic N-heterocycle. In a preferred embodiment, moiety L1 is an imidazole or triazole, most preferably an unsubstituted triazole.

Thus, in an embodiment the present compound may be of the general formulae (1a):

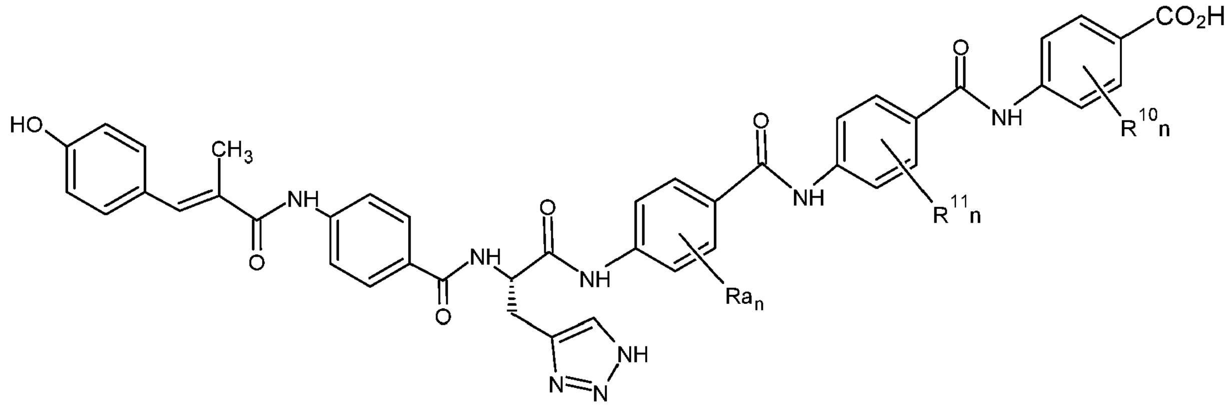

with BC, $R^a{}_n$, $R^{10}n$ and $R^{11}n$ as described previously above.

In another preferred embodiment, n of $R^{10}{}_n$ and n of $R^{11}{}_n$ being 0, 1, 2, 3 or 4, in particular n of $R^{10}{}_n$ and n of $R^{11}{}_n$ being 0, 1, 2 or 3, and with each $R^{10}$ and with each $R^{11}$ independently from any other $R^{10}$ being selected from -OH, $-OCH_3$, $-OC_2H_5$ or -OiPr, particularly with one $R^{10}$ or $R^{11}$ being -OH and the other $R^{10}$ or $R^{11}$ being $-OCH_3$, $-OC_2H_5$ or -OiPr respectively. In one specific embodiment R10 is H and R11 is one of from -OH, $-OCH_3$, $-OC_2H_5$ or -OiPr.

In an embodiment the present compound may be of the general formulae (2)

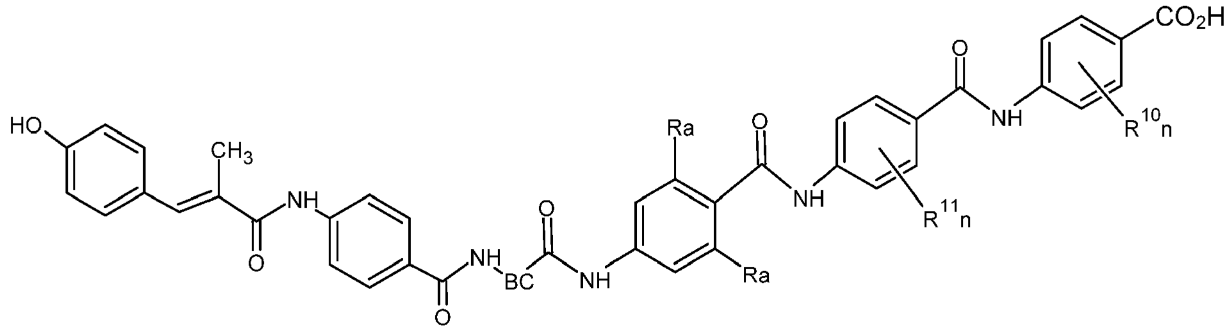

with $R^a$ being a substituent selected from halogen, in particular -Cl, - F, - Br, -I, $-NO_2$, - $NH_2$, $-CO_2H$, -CN, - $CF_3$, with BC, $R^{10}n$ and $R^{11}n$ as described previously above.

In another embodiment the present compound may be of the general formulae (3)

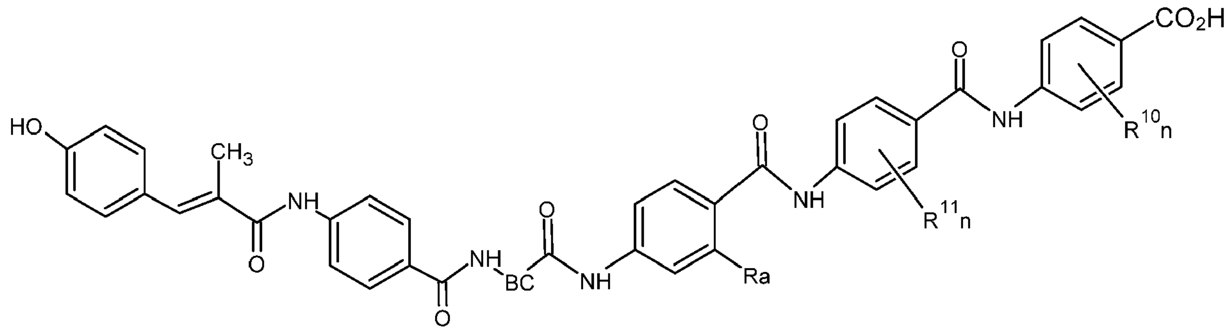

with $R^a$ being a substituent selected from halogen, in particular -Cl, - F, - Br, -I, $-NO_2$, - $NH_2$, $-CO_2H$, -CN, - $CF_3$, with BC, $R^{10}n$ and $R^{11}n$ as described previously above.

In a further embodiment the present compound may be of the general formulae (4)

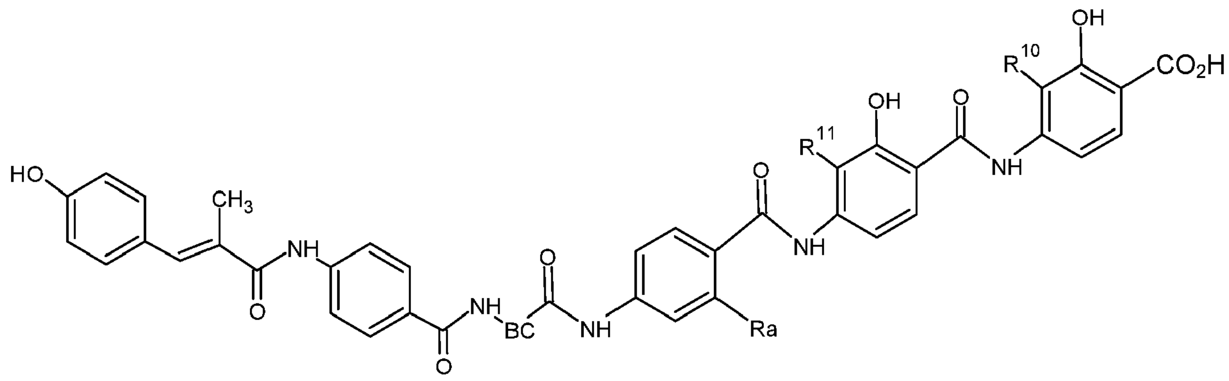

with $R^a$ being a substituent selected from halogen, in particular -Cl, - F, - Br, -I, $-NO_2$, - $NH_2$, $-CO_2H$, -CN, - $CF_3$, with BC, $R^{10}$ and $R^{11}$ as described previously above.

In still another embodiment the present compound may be of the general formulae (5)

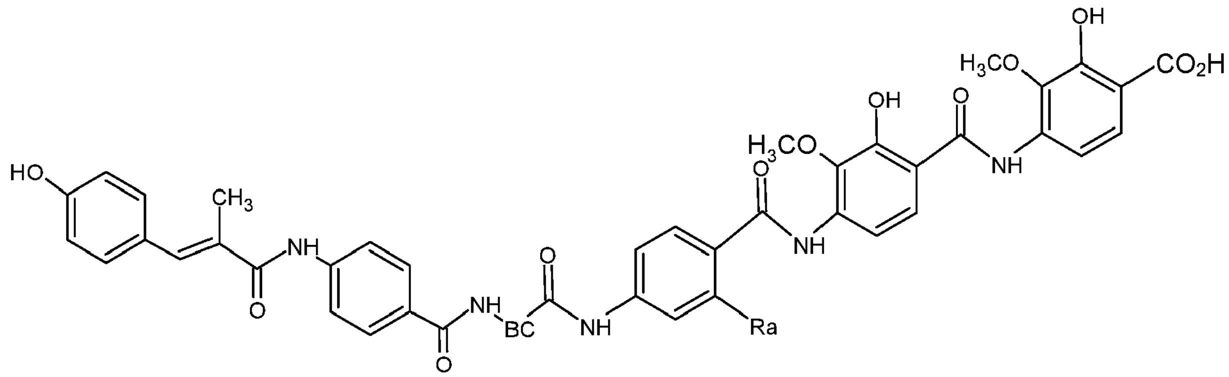

with $R^a$ being a substituent selected from halogen, in particular -Cl, - F, - Br, -I, $-NO_2$, - $NH_2$, $-CO_2H$, -CN, - $CF_3$, with BC as described previously above.

In still another embodiment the present compound may be of the general formulae (6)

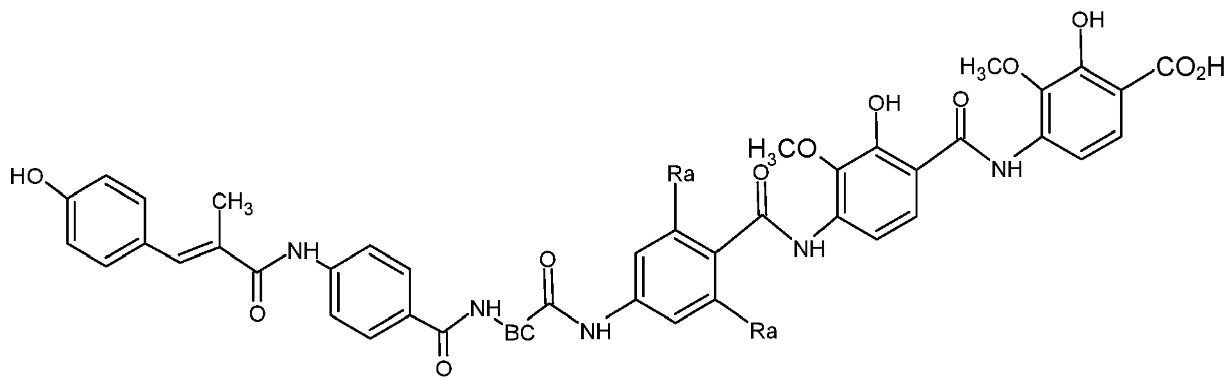

with $R^a$ being an electron withdrawing moiety selected from-Cl, - F, - Br, -I, - $CF_3$, with BC as described previously above.

Particular embodiments of the invention are one of the following compounds:

Compound 1:

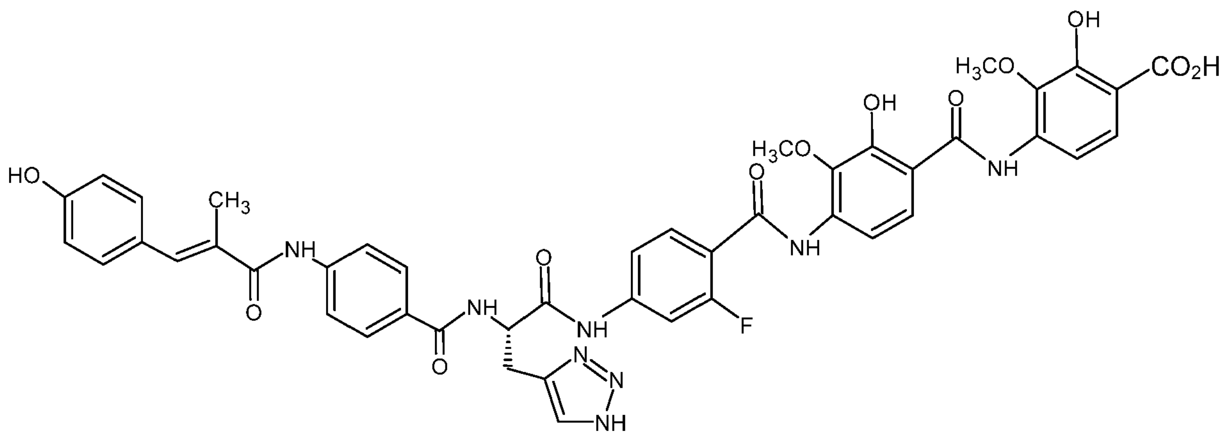

Compound 2:

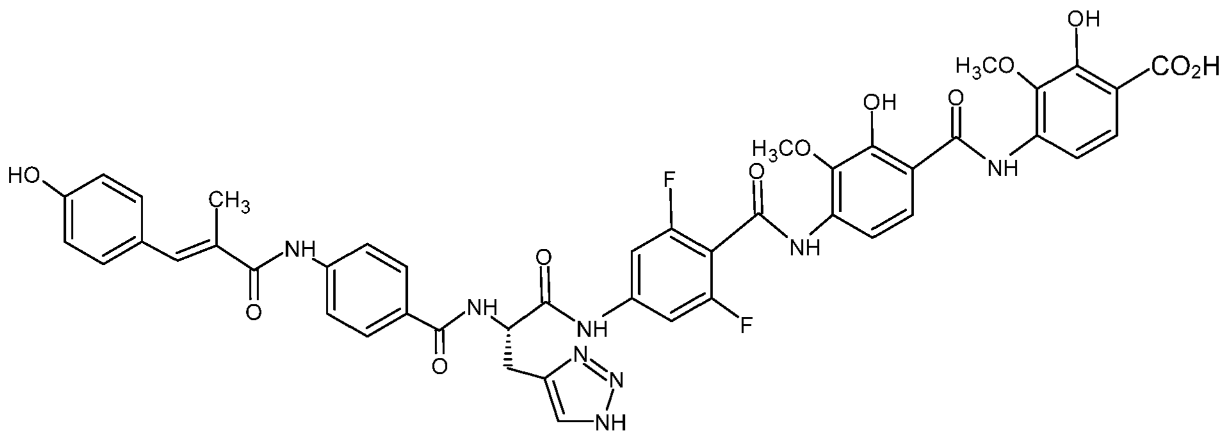

Compound 3:

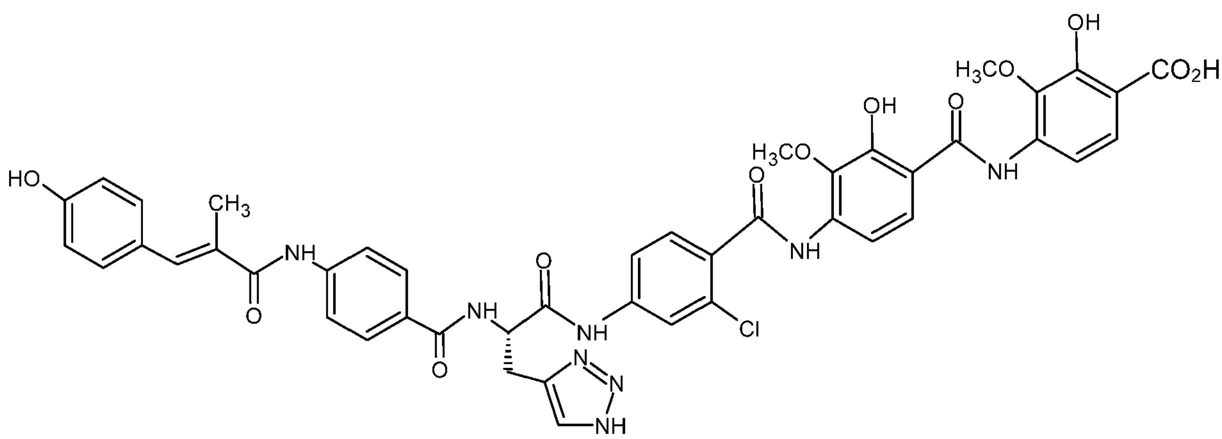

Compound 4:

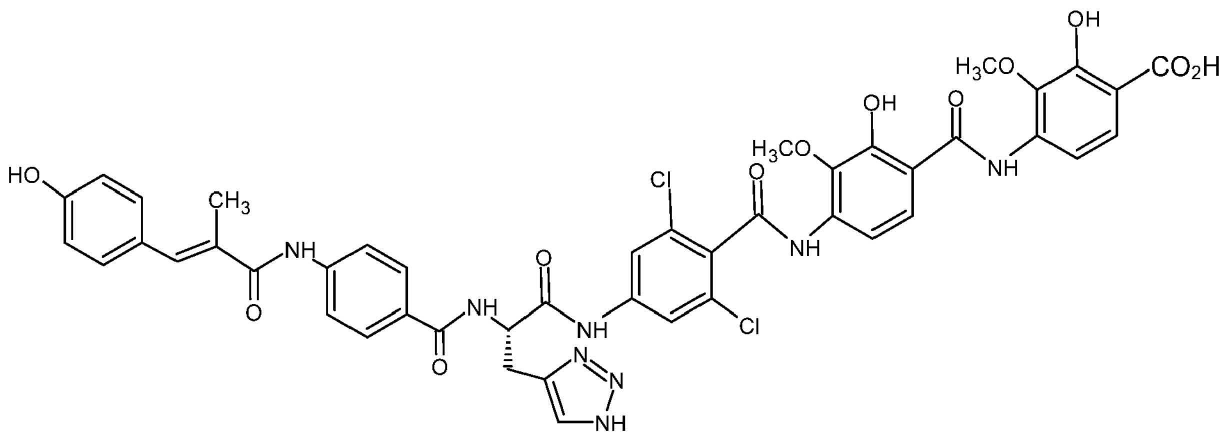

Compound 5:

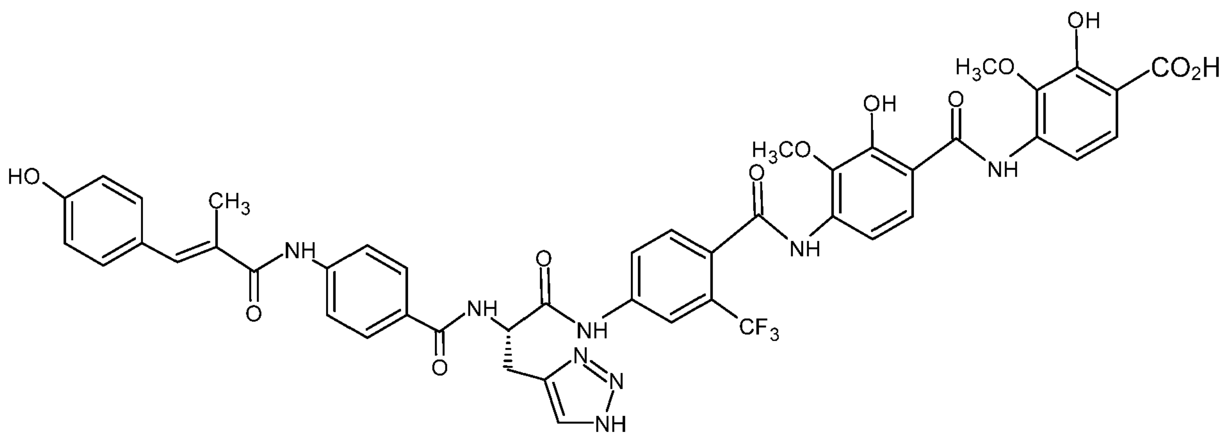

Compound 6:

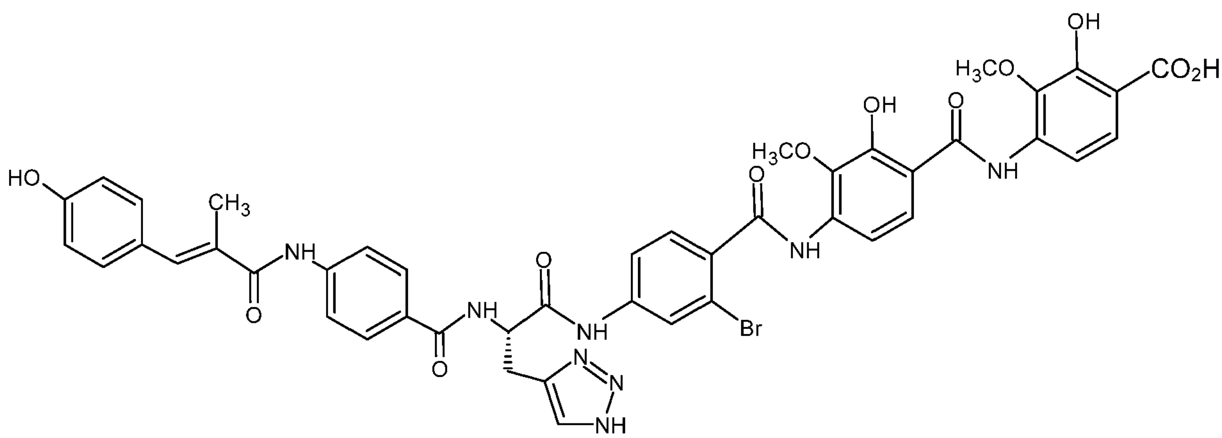

Compound 7:

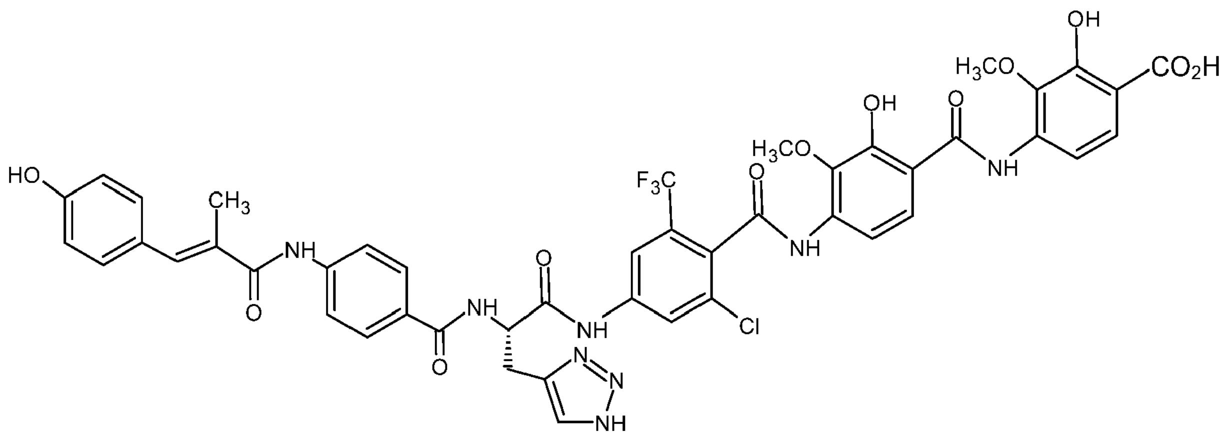

Compound 8:

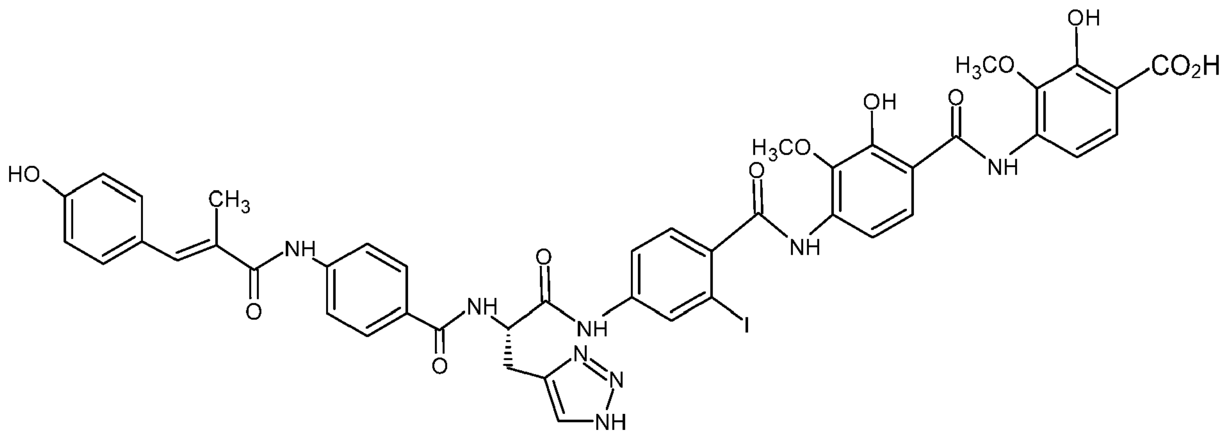

The compounds of the present invention may be used in a method of treatment of diseases, in particular for use in a method of treatment of bacterial infections caused by gram-negative or gram-positive bacterial strains.

The bacterial infection may be an infection (by a gram-negative bacterium) caused by one of the genus Acinetobacter, Bordatella, Borellia, Brucella, Camphylobacter, Chlamydia, Chlamydophila, Enterobacter, Escherichia, Francisella, Haemophilus, Helicobacter, Klebsiella, Legionella, Leptospira, Morganella Moraxella, Neisseria, Proteus, Pseudomonas, Rickettsia, Shigella, Salmonella, Stenotrophomonas, Treponema or Yersinia, in particular an infection caused by one of the genus Escherichia, Enterobacter, Salmonella, Klebsiella, Pseudomonas, Haemophilus, Shigella, Proteus or Morganella.

In a further embodiment, the bacterial infection is an infection caused

- by a gram-positive bacterium, particularly an infection by one of the genus Bacillus, Clostridium , Corynebacterium, Enterococcus, Listeria, Micrococcus, Staphylococcus or Streptococcus, further in particular by one of the genus of Staphylococcus, Streptococcus, Bacillus or Micrococcus or
- by a bacterium of the family of Mycobacteriaceae, in particular of the genus Mycobacterium, further in particular an infection by one of *Mycobacterium tuberculosis, Mycobacterium leprae, Mycobacterium ulcerans* or *Mycobacterium avium,* or
- by a bacterium of the family of Mycoplasmataceae, in particular of the genus Mycoplasma, further in particular an infection by *Mycoplasma pneumonia.*

For this purpose, the present compounds may be provided in a pharmaceutical acceptable form. Pharmaceutically acceptable salts of the present compounds mean both their organic and inorganic salts as described in Remington's Pharmaceutical Sciences (17th edition, page 1418 (1985)). Because of the physical and chemical stability and the solubility, preference is given for acidic groups inter alia to sodium, potassium, calcium and ammonium salts; preference is given for basic groups inter alia to salts of maleic acid, fumaric acid, succinic acid, malic acid, tartaric acid, methylsulfonic

acid, hydrochloric acid, sulfuric acid, phosphoric acid or of carboxylic acids or sulfonic acids, for example as hydrochlorides, hydrobromides, phosphates, sulfates, methanesulfonates, acetates, lactates, maleates, fumarates, malates, gluconates, and salts of amino acids, of natural bases or carboxylic acids. The preparation of pharmaceutically acceptable salts from compounds of the formula (I) which are capable of salt formation, including their stereoisomeric forms, takes place in a manner known per se. The present compounds form stable alkali metal, alkaline earth metal or optionally substituted ammonium salts with basic reagents such as hydroxides, carbonates, bicarbonates, alcoholates and ammonia or organic bases, for example trimethyl- or triethylamine, ethanolamine, diethanolamine or triethanolamine, trometamol or else basic amino acids, for example lysine, ornithine or arginine. Where the compounds of the formula (I) have basic groups, stable acid addition salts can also be prepared with strong acids. Suitable pharmaceutically acceptable acid addition salts of the compounds of the invention are salts of inorganic acids such as hydrochloric acid, hydrobromic, phosphoric, metaphosphoric, nitric and sulfuric acid, and of organic acids such as, for example, acetic acid, benzenesulfonic, benzoic, citric, ethanesulfonic, fumaric, gluconic, glycolic, isethionic, lactic, lactobionic, maleic, malic, methanesulfonic, succinic, p-toluenesulfonic and tartaric acid. The hydrochloride salt is a preferred salt.

In a preferred embodiment formulations of the present albicidin derivatives are provided which contain cyclodextrins for improving solubility of the otherwise poorly soluble albicidin derivatives. Cyclodextrins are used in a concentration of 20-40%, preferably 25-35 %, more preferably 28-30%.

Salts with a pharmaceutically unacceptable anion such as, for example, trifluoroacetate likewise belong within the framework of the invention as useful intermediates for the preparation or purification of pharmaceutically acceptable salts and/or for use in non-therapeutic, for example in vitro, applications.

The present invention furthermore relates to pharmaceutical preparations (or pharmaceutical compositions) which contain an effective amount of at least one of the present compounds and/or its pharmaceutically acceptable salts and a pharmaceutically acceptable carrier, i. e. one or more pharmaceutically acceptable carrier substances (or vehicles) and/or additives (or excipients). The pharmaceuticals can be administered orally, for example in the form of pills, tablets, lacquered tablets, coated tablets, granules, hard and soft gelatine capsules, solutions, syrups, emulsions, suspensions or aerosol mixtures. Administration, however, can also be carried out rectally, for example in the form of suppositories, or parenterally, for example intravenously, intramuscularly or subcutaneously, in the form of injection solutions or infusion solutions, microcapsules, implants or rods, or percutaneously or topically, for example in the form of ointments, solutions or tinctures, or in other ways, for example in the form of aerosols or nasal sprays.

The pharmaceutical preparations according to the invention are prepared in a manner known per se and familiar to one skilled in the art, pharmaceutically acceptable inert inorganic and/or organic carrier substances and/or additives being used in addition to the compound(s) of the formula (I) and/or its (their) pharmaceutically acceptable salts and/or its (their) prodrugs. For the production of pills, tablets, coated tablets and hard gelatine capsules it is possible to use, for example, lactose, corn starch or derivatives thereof, talc, stearic acid or its salts, etc. Carrier substances for soft gelatine capsules and suppositories are, for example, fats, waxes, semisolid and liquid polyols, natural or hardened oils, etc. Suitable carrier substances for the production of solutions, for example injection solutions, or of emulsions or syrups are, for example, water, saline, alcohols, glycerol, polyols, sucrose, invert sugar, glucose, vegetable oils, etc. Suitable carrier substances for microcapsules, implants or rods are, for example, copolymers of glycolic acid and lactic acid. The pharmaceutical preparations normally contain about 0.5 to about 90 % by weight of the present compounds and/or their pharmaceutically acceptable salts and/or their prodrugs. The amount of the active ingredient of the formula (I) and/or its pharmaceutically acceptable salts and/or its prodrugs in the pharmaceutical preparations normally is from about 0.5 to about 1000 mg, preferably from about 1 to about 500 mg.

A prodrug is a precursor chemical compound of a biological active compound of the present invention. Instead of administering the active compound or drug, a prodrug might be used instead to improve the absorption, distribution, metabolization and excretion. Prodrugs are often designed to improve bioavailability when a drug itself is poorly absorbed from the gastrointestinal tract. A prodrug may also be used to improve the selectively of the drug. This reduces adverse or unintended effects of a drug, especially important in treatments like chemotherapy, which can have severe unintended and undesirable side effects.

In addition to the active compound according to the invention and/or their pharmaceutically acceptable salts and to carrier substances, the pharmaceutical preparations can contain one or more additives such as, for example, fillers, disintegrants, binders, lubricants, wetting agents, stabilizers, emulsifiers, preservatives, sweeteners, colorants, flavourings, aromatizers, thickeners, diluents, buffer substances, solvents, solubilizers, agents for achieving a depot effect, salts for altering the osmotic pressure, coating agents or antioxidants. They can also contain two or more of the present compounds and/or their pharmaceutically acceptable salts. In case a pharmaceutical preparation contains two or more of the present compounds the selection of the individual compounds can aim at a specific overall pharmacological profile of the pharmaceutical preparation. For example, a highly potent compound with a shorter duration of action may be combined with a long-acting compound of lower potency. The flexibility permitted with respect to the choice of substituents in the present compounds allows a great deal of control over the biological and physico-chemical properties of the compounds and thus allows the selection of such desired compounds. Furthermore, in addition to at least one

compound and/or its pharmaceutically acceptable salts, the pharmaceutical preparations can also contain one or more other therapeutically or prophylactically active ingredients. When using the present compounds the dose can vary within wide limits and, as is customary and is known to the physician, is to be suited to the individual conditions in each individual case. It depends, for example, on the specific compound employed, on the nature and severity of the disease to be treated, on the mode and the schedule of administration, or on whether an acute or chronic condition is treated or whether prophylaxis is carried out. An appropriate dosage can be established using clinical approaches well known in the medical art. In general, the daily dose for achieving the desired results in an adult weighing about 75 kg is from about 0.01 to about 100 mg/kg, preferably from about 0.1 to about 50 mg/kg, in particular from about 0.1 to about 10 mg/kg, (in each case in mg per kg of body weight). The daily dose can be divided, in particular in the case of the administration of relatively large amounts, into several, for example 2, 3 or 4, part administrations. As usual, depending on individual behaviour it may be necessary to deviate upwards or downwards from the daily dose indicated.

The compounds of the invention may also exist in various polymorphous forms, for example as amorphous and crystalline polymorphous forms. All polymorphous forms of the compounds of the invention belong within the framework of the invention and are a further aspect of the invention.

The compounds of the present invention may be present as optical isomers or as mixtures thereof. The invention relates both to the pure isomers and all possible isomeric mixtures and is hereinafter understood as doing so, even if stereochemical details are not specifically mentioned in every case. Enantiomeric mixtures of compounds of the general formula 1, which are obtainable by the process or any other way, may be separated in known manner - on the basis of the physical-chemical differences of their components - into pure enantiomers, for example by fractional crystallisation, distillation and/or chromatography, in particular by preparative HPLC using a chiral HPLC column.

According to the invention, apart from separation of corresponding isomer mixtures, generally known methods of diastereoselective or enantioselective synthesis can also be applied to obtain pure diastereoisomers or enantiomers, e.g. by carrying out the method described hereinafter and using educts with correspondingly suitable stereochemistry.

It is advantageous to isolate or synthesise the biologically more active isomer, provided that the individual compounds have different biological activities.

The present invention is explained in more detail by means of the following examples.

**Methods of synthesis**

One general procedure for the synthesis of albicidin-derivatives with variations to amide bonds may comprise the steps according to the following procedure.

Compound 3

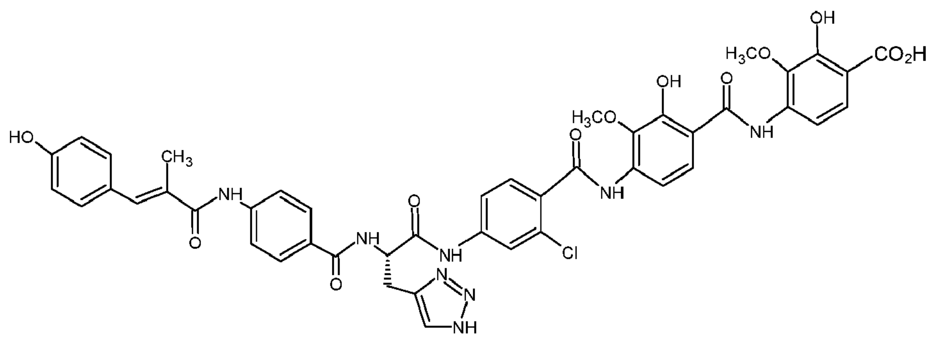

Compound 3 is synthesized in a multistep synthesis route as follows:

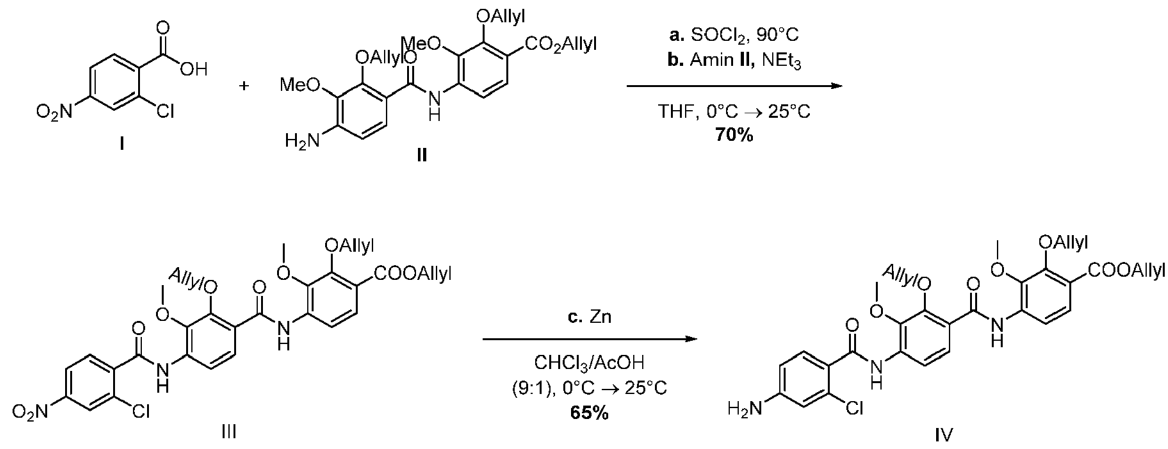


Preparation of compound III:

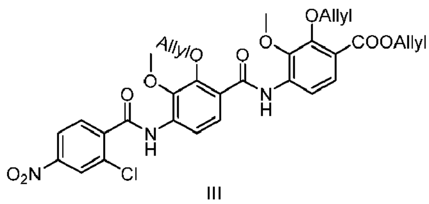

III

The commercially available 2-Chloro-4-nitrobenzoeic acid I (348 mg, 743 μmol, 1.40 eq.) was dissolved in $SOCl_2$ (5 mL) and refluxed for 2 h. All volatiles were removed and the residue dissolved in THF (5 mL). The prepared acid chloride solution was added at -15°C to a prepared solution of the literature known amine II (348 mg, 743 μmol, 1.00 eq.) in anhydrous THF (24 mL) and triethylamine (207 μL, 1.49 mmol, 2.00 eq.). The reaction mixture was stirred for 20 minutes and diluted with diethyl ether (22 ml). The solid was filtered, washed with diethyl ether (3 x 50 ml) and dried *in vacuo* to yield III (338 mg, 70%) as a yellow solid.

**$^1$H NMR** (500 MHz, DMSO-$d_6$) δ 10.62 (s, 1H), 10.53 (s, 1H), 8.41 (d, *J* = 2.2 Hz, 1H), 8.35-8.27 (m, 2H), 8.11 (d, *J*= 8.8 Hz, 1H), 7.90 (d, *J* = 8.4 Hz, 1H), 7.83 (d, *J* = 8.8 Hz, 1H), 7.57 (d, *J* = 8.8 Hz, 1H), 6.06 (dddt, *J* = 27.9, 16.1, 10.7, 5.3 Hz, 3H), 5.40 (ddd, *J* = 18.2, 16.1, 2.1 Hz, 3H), 5.31 - 5.21 (m, 3H), 4.78 (dd, *J* = 11.3, 5.9 Hz, 4H), 4.54 (d, *J* = 5.8 Hz, 2H), 3.93 (s, 3H), 3.87 (s, 3H).

**$^{13}$C NMR** (126 MHz, DMSO-$d_6$) δ 164.4, 151.0, 149.7, 148.3, 142.5, 136.4, 135.8, 133.9, 132.7, 132.6, 131.1, 130.2, 126.2, 125.7, 124.4, 122.3, 120.3, 120.3, 120.1, 118.1, 117.8, 114.8, 75.1, 74.5, 65.1, 61.1, 61.0.

**HRMS (ESI-ORBITRAP):** *m/z* $[M+H]^+$ ber. für $C_{32}H_{31}ClN_3O_{10}$: 652.1692; gef. 652.1696.

Preparation of compound **IV:**

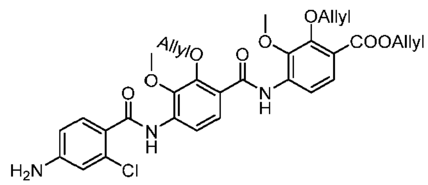

**IV**

Compound III (331 mg, 508 μmol, 1.00 eq.) was suspended in a mixture of chloroform (90 ml) and acetic acid (10 ml) and cooled to 0 °C. Zinc dust (664 mg, 10.2 mmol, 20.0 eq.) was added portion wise. After 20 min the reaction was proven to be complete (verified by TLC-control). The solid was filtered and washed with DCM (3 x 100 ml). The combined liquids were evaporated to dryness. The residue was taken up in DCM (300 ml) and saturated aqueous

$NaHCO_3$-Solution (300 ml). The aqueous phase was further extracted twice with DCM (2 x 100 ml). The combined organic fractions were washed successively with saturated aqueous $NaHCO_3$-Solution (1 × 30 ml), distilled water (1 × 30 ml) and brine (1 × 30 ml), dried over $Na_2SO_4$ and evaporated to obtain **IV** (206 mg, 65%) as a yellow solid.

**$^1$H NMR** (400 MHz, DMSO-$d_6$) δ 10.63 (s, 1H), 9.61 (s, 1H), 8.36 - 8.29 (m, 1H), 8.17 (d, *J* = 8.9 Hz, 1H), 7.81 (d, *J* = 8.9 Hz, 1H), 7.57 (d, *J* = 8.8 Hz, 1H), 7.51 (d, *J* = 8.5 Hz, 1H), 6.67 (d, *J* = 2.2 Hz, 1H), 6.59 (dd, *J* = 8.5, 2.2 Hz, 1H), 6.17 - 6.08 (m, 2H), 6.08 - 5.96 (m, 3H), 5.40 (ddq, *J* = 17.2, 12.1, 1.7 Hz, 3H), 5.32 - 5.20 (m, 3H), 4.78 (ddt, *J* = 7.0, 5.5, 1.3 Hz, 4H), 4.54 (dt, *J* = 5.7, 1.4 Hz, 2H), 3.92 (s, 3H), 3.91 (s, 3H).

**($^1$H,$^{13}$C)-HSQC_ed NMR** (101 MHz, DMSO-$d_6$) δ 115.3, 116.9, 126.5, 126.6, 126.8, 132.9, 114.2, 112.6, 133.8, 119.4, 119.8, 119.5, 118.6, 75.5, 65.5, 75.0, 61.4.

**HRMS (ESI-ORBITRAP):** *m/z* $[M+H]^+$ ber. für $C_{32}H_{33}ClN_3O_8$: 622.1951; gef. 622.1952.

Preparation of compound **VI:**

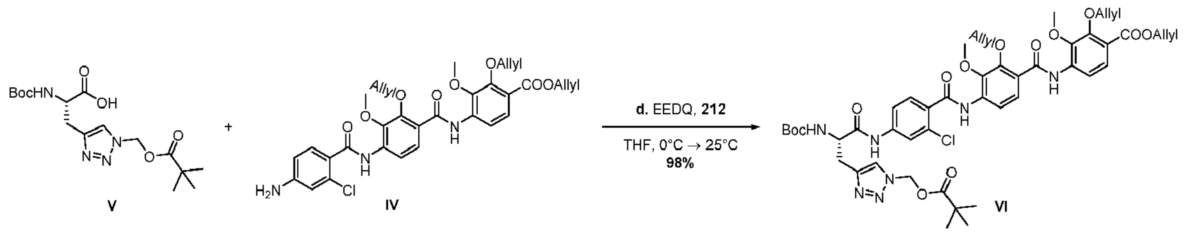


Literature known Boc-β-(1-pivaloyloxymethyl)-1,2,3-triazol-4-yl)-Alanine **V** (237 mg, 640 μmol, 2.00 eq.) was dissolved in THF (20 ml) and cooled to 0 °C. *N*-Ethoxycarbonyl-2-ethoxy-1,2-dihydroqinoline (EEDQ) (237 mg, 960 μmol, 3.00 eq.) was added and after 5 minutes compound **IV** (199 mg, 320 μmol, 1.00 eq.) was added. The reaction mixture was slowly warmed to room temperature and stirred for 16 h. All volatiles were removed *in vacuo* and the residue was taken up in ethyl acetate (100 ml). The organic fraction was washed with saturated aqueous $NaHCO_3$-Solution (3 x 50 ml) and brine (1 × 50 ml), dried over $Na_2SO_4$ and evaporated. The residue was purified *via* flash chromatography on silica gel eluting with 1-15% acetone in DCM. Compound **VI** (305 mg, 98%) was obtained as a colourless solid.

**$^1$H NMR** (500 MHz, DMSO-$d_6$) δ 10.63 (s, 1H), 10.45 (s, 1H), 9.99 (s, 1H), 8.33 (d, *J* = 8.8 Hz, 1H), 8.08 (d, *J* = 9.2 Hz, 1H), 7.98 (s, 1H), 7.91 (d, *J* = 1.9 Hz, 1H), 7.81 (d, *J* = 8.9 Hz, 1H), 7.61 (d, *J* = 8.4 Hz, 1H), 7.57 (d, *J* = 8.7 Hz, 2H), 7.22 (d, *J* = 7.9 Hz, 1H), 6.29 (s, 2H), 6.07 (dddd, *J* = 33.5, 22.5, 10.4, 4.6 Hz, 3H), 5.45 - 5.36 (m, 3H), 5.30 - 5.21 (m, 3H), 4.78 (t, *J* = 7.4 Hz, 4H), 4.54 (d, *J* = 5.7 Hz, 2H), 4.37 (d, *J* = 6.2 Hz, 1H), 3.93 (s, 3H), 3.89 (s, 3H), 3.15 -3.08 (m, 1H), 3.00 (dd, *J* = 14.7, 9.2 Hz, 1H), 1.36 (s, 9H), 1.10 (s, 9H).

**$^{13}$C NMR** (126 MHz, DMSO-$d_6$) δ 115.6, 119.0, 124.9, 120.3, 126.7, 131.2, 119.0, 126.7, 69.9, 134.9, 119.8, 119.0, 75.9, 65.8, 75.2, 61.6, 61.7, 28.5, 26.9.

**HRMS (ESI-ORBITRAP):** *m/z* $[M+H]^+$ ber. für $C_{48}H_{57}ClN_7O_{13}$: 974.3697; gef. 974.3688.

Preparation of compound **VII**:

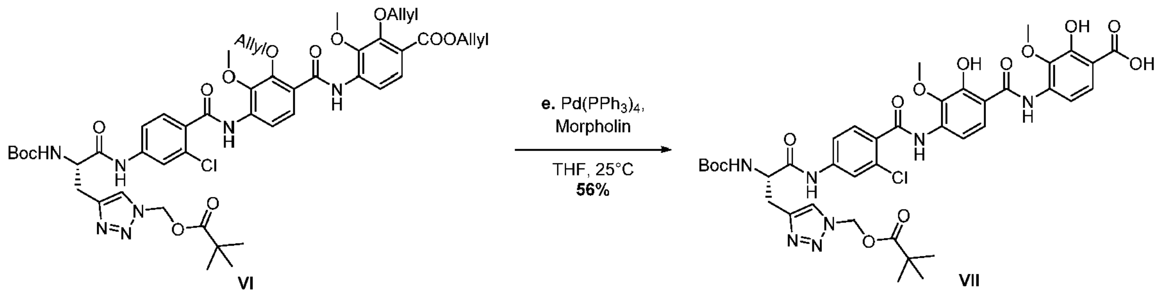


Tetrapeptide **VI** (220 mg, 226 μmol, 1.00 eq.) was dissolved in THF (5 ml) and morpholine (390 μL mL, 4.52 mmol, 20.0 eq.) and *tetrakis*(triphenylphosphin)palladium(0) (104 mg, 90.3 μmol, 0.40 eq.) were added. The mixture was stirred for 2.5 h shielded from light. All volatiles were removed *in vacuo* and the residue was purified via flash

chromatography on C-18-material eluting with 5 to 50% acetonitrile in water. Compound **VII** (108 mg, 56%) was obtained as a white solid.

**$^1$H NMR** (500 MHz, DMSO-$d_6$) δ 11.57 (s, 1H), 10.91 (s, 1H), 10.45 (s, 1H), 9.93 (s, 1H), 7.98 (s, 1H), 7.90 (d, *J* = 1.9 Hz, 1H), 7.81 (d, *J* = 8.9 Hz, 1H), 7.74 (s, 2H), 7.60 (d, *J* = 8.4 Hz, 1H), 7.56 (dd, *J* = 8.4, 1.9 Hz, 1H), 7.48 (d, *J* = 8.6 Hz, 1H), 7.22 (d, *J* = 7.9 Hz, 1H), 6.29 (s, 2H), 4.42 - 4.32 (m, 1H), 3.87 (s, 3H), 3.77 (s, 3H), 3.12 (dd, *J* = 14.7, 5.0 Hz, 1H), 3.00 (dd, *J* = 14.8, 9.3 Hz, 1H), 1.36 (s, 9H), 1.10 (s, 9H).

**($^1$H,$^{13}$C)-HSQC_ed NMR** (126 MHz, DMSO-$d_6$) δ 124.6, 120.0, 125.4, 130.7, 118.0, 125.4, 70.2, 60.2, 61.2, 28.7, 26.9.

**HRMS (ESI-ORBITRAP):** *m/z* $[M+H]^+$ ber. für $C_{39}H_{45}ClN_7O_{13}$: 854.2758; gef. 854.2758.

Preparation of compound **VIII:**

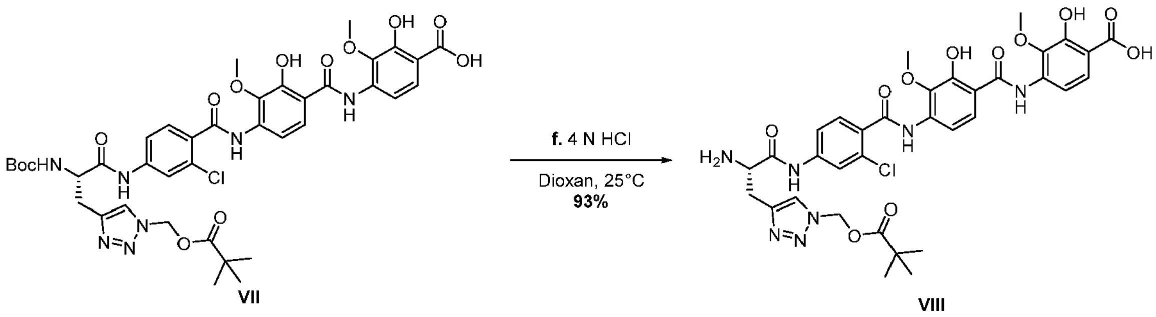


Tetrapeptide **VII** (108 mg, 126 μmol, 1.00 eq.) was dissolved 4 N HCl in dioxane and stirred for 1 hour. The solvent was evaporated in vacuo and the product **VIII** (94 mg, 93%) was obtained as white solid.

**$^1$H NMR** (500 MHz, DMSO-$d_6$) δ 11.53 (s, 1H), 11.16 (s, 1H), 10.02 (s, 1H), 8.48 (s, 2H), 8.14 (s, 1H), 8.04 (d, *J* = 8.9 Hz, 1H), 7.87 - 7.80 (m, 2H), 7.76 (s, 1H), 7.63 (d, *J* = 8.4 Hz, 1H), 7.57 (dd, *J* = 16.8, 9.0 Hz, 2H), 6.29 (s, 2H), 6.26 (d, *J* = 4.7 Hz, 1H), 4.33 (d, *J* = 13.4 Hz, 1H), 3.91 (s, 3H), 3.77 (s, 3H), 3.69 (dd, *J* = 16.5, 5.2 Hz, 1H), 3.50-3.46 (m, 1H), 1.10 (s, 9H).

**($^1$H,$^{13}$C)-HSQC_ed NMR** (126 MHz, DMSO-$d_6$) δ 125.5, 124.4, 109.3, 119.1, 125.5, 95.7, 71.1, 61.3, 61.8, 26.6.

**HRMS (ESI-ORBITRAP):** *m/z* $[M+H]^+$ ber. für $C_{34}H_{37}ClN_7O_{11}$: 754.2234; gef. 754.2230.

Preparation of compound 3:

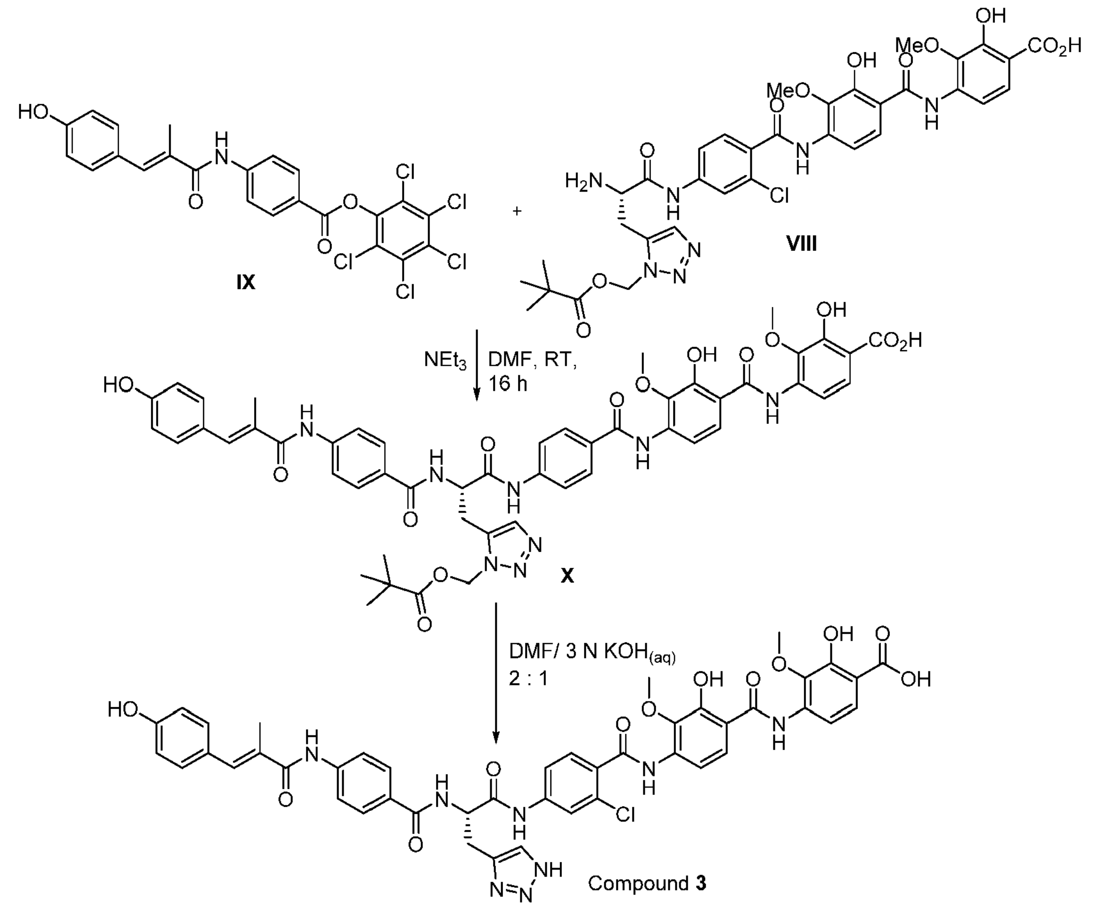


IX

VIII

X

Compound **3**

The tetrapeptide VIII (45.0 mg, 60 μmol, 1.00 eq.) was dissolved in DMF (2 ml) and triethylamine (83 μL, 10.0 eq.) was added. After adding the literature known active ester IX (39 mg, 65.6 μmol, 1.10 eq.), the mixture was stirred for 16 h. Then cooled to 0 °C and 3 N $KOH_{(aq)}$ (1 ml) was added dropwise. After 15 min of stirring, 550 μl of 6 N $HCl_{(aq)}$ were added dropwise. The resulting mixture was evaporated to dryness. The residue was purified *via* prep HPLC. Compound 3 (10.4 mg, 18%) was obtained as a white fluffy solid.

**RP-HPLC:** 38-52% MeCN (+ 0.1% TFA) in milliQ $H_2O$ (+0.1% TFA); 30 min; 60 mL/min. $[a]_D^{24}$ = +32° (c 0.08, DMSO).

**$^1$H NMR** (700 MHz, DMSO-*d*$_6$) δ 11.57 (s, 1H), 11.52 (d, *J* = 18.0 Hz, 1H), 11.18 (s, 1H), 10.53 (s, 1H), 10.08 (s, 1H), 10.02 (d, *J* = 5.9 Hz, 1H), 9.76 (s, 1H), 8.71 (d, *J* = 7.6 Hz, 1H), 8.06 (dd, *J* = 8.9, 5.2 Hz, 1H), 7.92 (d, *J* = 1.7 Hz, 1H), 7.87 (d, *J* = 8.6 Hz, 2H), 7.84 - 7.79 (m, 3H), 7.76 (s, 1H), 7.67 (s, 1H), 7.63 - 7.57 (m, 3H), 7.35 (d, *J* = 8.5 Hz, 2H), 7.26 (s, 1H), 6.84 (d, *J* = 8.3 Hz, 2H), 4.87 (q, *J* = 7.3 Hz, 1H), 3.91 (s, 3H), 3.76 (s, 3H), 3.29 (dd, *J* = 14.7, 5.8 Hz, 1H), 3.23 (dd, *J* = 14.8, 9.0 Hz, 1H), 2.11 (d, *J* = 1.3 Hz, 3H).

**($^1$H,$^{13}$C)-HSQC_ed NMR** (176 MHz, DMSO-*d*$_6$) δ 110.7, 120.0, 128.7, 128.7, 119.5, 126.2, 114.1, 130.5, 118.0, 126.2, 131.8, 134.4, 115.8, 54.8, 60.7, 61.3, 15.0.

**HRMS (ESI-ORBITRAP):** *m/z* $[M+H]^+$ ber. für $C_{45}H_{40}ClN_8O_{12}$: 919.2449, gef. 919.2450, $R_T$ = 8.26 min.

The following compounds are obtained in analog synthesis procedures.

Compound 1

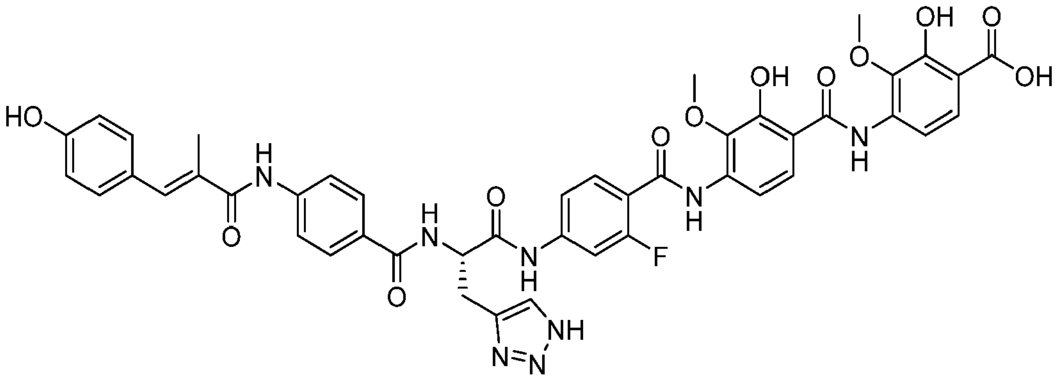

Chemical Formula: $C_{45}H_{39}FN_8O_{12}$
Exact Mass: 902.2671

**RP-HPLC:** 35-58% MeCN (+ 0.1% TFA) in milliQ $H_2O$ (+0.1% TFA); 35 min; 70 mL/min. $[a]_D^{24}$ = +27.0° (c 0.11, DMSO).

$^1$**H NMR** (500 MHz, DMSO-$d_6$) δ 11.64 (s, 1H), 11.11 (s, 1H), 10.71 (s, 1H), 10.07 (s, 1H), 9.76 (s, 1H), 9.61 (d, $J$ = 9.6 Hz, 1H), 8.72 (s, 1H), 8.09 - 7.76 (m, 10H), 7.68 (s, 1H), 7.57 (d, $J$ = 8.7 Hz, 1H), 7.53 - 7.47 (m, 1H), 7.35 (d, $J$ = 8.8 Hz, 2H), 7.26 (s, 1H), 6.87 - 6.81 (m, 2H), 4.89 (dd, $J$ = 14.9, 5.0 Hz, 1H), 3.91 (s, 3H), 3.82 (s, 3H), 3.23 (d, $J$ = 13.8 Hz, 2H), 2.11 (s, 3H).

$^{19}$**F NMR** (471 MHz, DMSO-$d_6$) δ -111.10 (d, $J$ = 8.9 Hz).

**($^1$H,$^{13}$C)-HSQC_ed NMR** (126 MHz, DMSO-$d_6$) δ 110.6, 112.7, 132.3, 128.6, 119.5, 106.6, 106.4, 126.0, 115.7, 131.8, 134.4, 115.9, 54.8, 60.6, 61.1, 40.3, 15.0.

**HRMS (ESI-ORBITRAP):** $m/z$ [M+H]$^+$ ber. für $C_{45}H_{40}FN_8O_{12}$: 903.2744, gef. 903.2746, $R_T$ = 8.19 min.

Compound 2

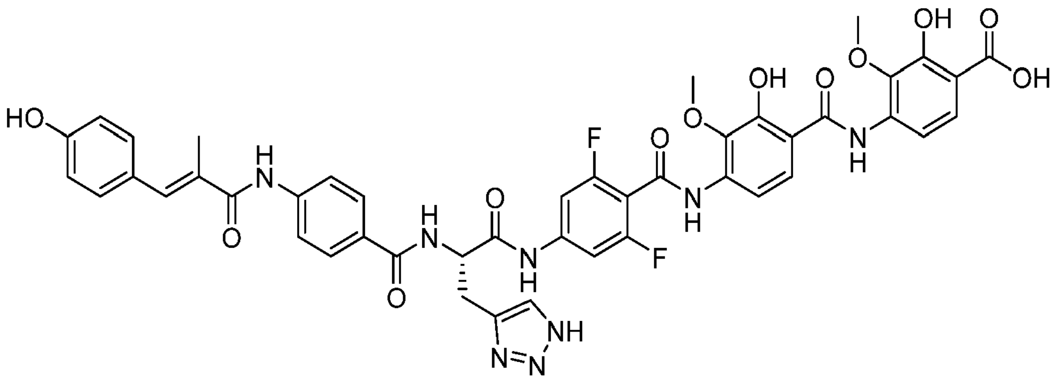

Chemical Formula: $C_{45}H_{38}F_2N_8O_{12}$
Exact Mass: 920.2577

**RP-HPLC:** 40-53% MeCN (+ 0.1% TFA) in milliQ $H_2O$ (+0.1% TFA); 30 min; 60 mL/min. $[a]_D^{24}$ = +85° (c 0.07, DMSO).

$^1$**H NMR** (500 MHz, DMSO-$d_6$) δ 11.52 (d, $J$ = 12.6 Hz, 1H), 11.13 (d, $J$ = 28.8 Hz, 1H), 10.69 (s, 1H), 10.35 (s, 1H), 10.07 (s, 1H), 9.75 (s, 1H), 8.73 (s, 1H), 8.04 (d, $J$ = 8.8 Hz, 1H), 7.87 (d, $J$ = 8.7 Hz, 2H), 7.84 - 7.79 (m, 3H), 7.76 (s, 1H), 7.64 (s, 1H), 7.59 (d, $J$ = 8.8 Hz, 1H), 7.45 (d, $J$ = 10.6 Hz, 2H), 7.35 (d, $J$ = 8.3 Hz, 2H), 7.26 (s, 1H), 6.89 - 6.77 (m, 2H), 4.90 - 4.82 (m, 1H), 3.91 (s, 3H), 3.74 (s, 3H), 3.27 - 3.18 (m, 2H), 2.11 (s, 3H).

$^{19}$**F NMR** (471 MHz, DMSO-$d_6$) δ -112.8.

**($^1$H,$^{13}$C)-HSQC_ed NMR** (176 MHz, DMSO-$d_6$) δ 110.6, 128.7, 119.5, 126.3, 119.5, 113.9, 119.0, 133.3, 126.2,

102.6, 131.8, 134.4, 129.6, 115.9, 115.6, 54.8, 60.6, 61.1, 27.9, 15.0, 22.5, 29.3.

**HRMS (ESI-ORBITRAP):** *m/z* $[M+H]^+$ ber. für $C_{45}H_{39}F_2N_8O_{12}$: 921.2650, gef. 921.2683, $R_T$ = 8.09 min.

Compound 5

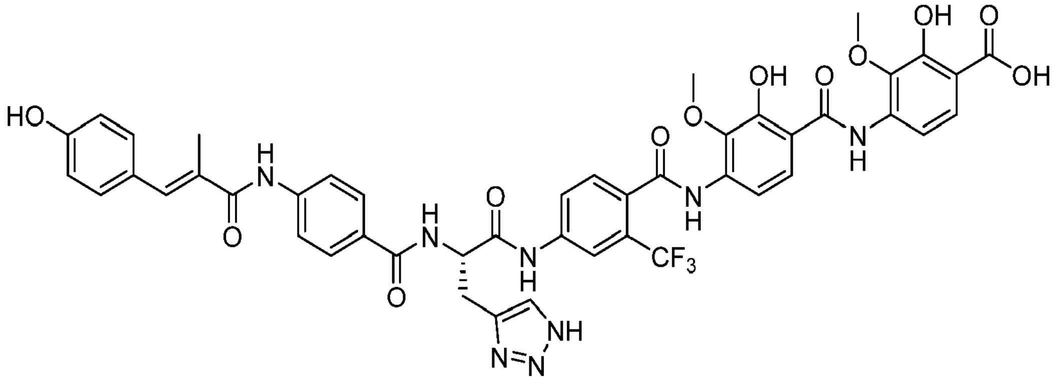

Chemical Formula: $C_{46}H_{39}F_3N_8O_{12}$
Exact Mass: 952.2640

**$^1$H NMR** (500 MHz, DMSO-$d_6$) δ 11.49 (d, *J* = 12.5 Hz, 1H), 11.17 (s, 1H), 10.64 (s, 1H), 10.15 (s, 1H), 10.07 (s, 1H), 9.75 (s, 1H), 8.71 (d, *J* = 7.4 Hz, 1H), 8.16 (d, *J* = 1.8 Hz, 1H), 8.05 (d, *J* = 8.8 Hz, 1H), 7.93 (d, *J* = 8.5 Hz, 1H), 7.87 (d, *J* = 8.6 Hz, 2H), 7.82 (d, *J* = 8.6 Hz, 3H), 7.68 (d, *J* = 8.3 Hz, 2H), 7.59 (d, *J* = 8.9 Hz, 1H), 7.35 (d, *J* = 8.3 Hz, 2H), 7.26 (s, 1H), 6.84 (d, *J*= 8.1 Hz, 2H), 4.89 (s, 1H), 3.91 (s, 3H), 3.74 (s, 3H), 3.26 (d, *J* = 8.9 Hz, 2H), 2.11 (s, 3H).

**$^{19}$F NMR** (659 MHz, DMSO-$d_6$) δ -58.10.

**($^1$H,$^{13}$C)-HSQC_ed NMR** (176 MHz, DMSO-$d_6$) δ 116.9, 110.6, 122.6, 119.5, 128.7, 128.7, 119.5, 126.2, 130.2, 114.4, 126.2, 131.8, 134.4, 115.8, 54.7, 60.7, 61.1, 27.8, 15.0, 29.4. **HRMS (ESI-ORBITRAP):** *m/z* $[M+H]^+$ calcd. $C_{46}H_{40}F_3N_8O_{12}$: 953.2712, gef. 953.2717, $R_T$ = 8.35 min.

Compound 6

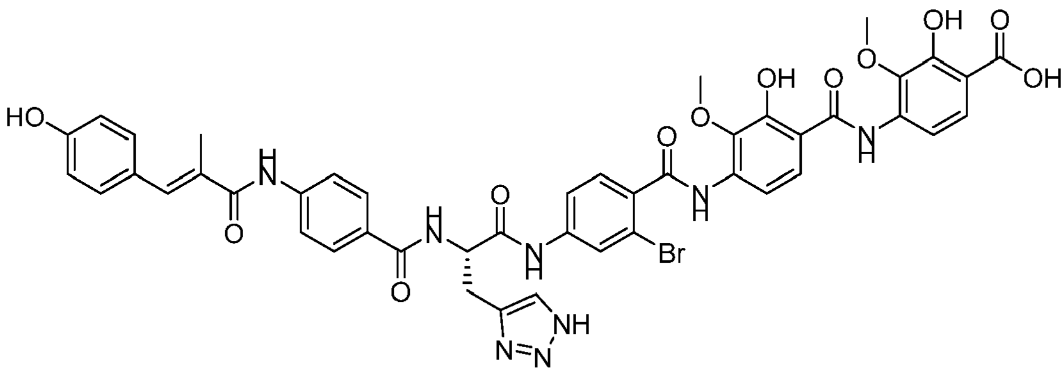

**$^1$H NMR** (500 MHz, DMSO-$d_6$) δ 11.58 (s, 1H), 11.52 (s, 1H), 11.17 (s, 1H), 10.49 (s, 1H), 10.07 (s, 1H), 10.00 (s, 1H), 9.75 (s, 1H), 8.69 (d, *J* = 7.4 Hz, 1H), 8.09 - 8.02 (m, 2H), 7.87 (d, *J* = 8.6 Hz, 2H), 7.82 (d, *J* = 8.4 Hz, 3H), 7.75 (s, 1H), 7.68 (s, 1H), 7.65 - 7.62 (m, 1H), 7.57 (dd, *J* = 19.5, 8.6 Hz, 2H), 7.35 (d, *J* = 8.3 Hz, 2H), 7.26 (s, 1H), 6.84 (d, *J* = 8.3 Hz, 2H), 4.87 (d, *J* = 7.6 Hz, 1H), 3.91 (s, 3H), 3.77 (s, 3H), 3.30 - 3.22 (m, 2H), 2.11 (s, 3H).

**HRMS (ESI-ORBITRAP):** *m/z* $[M+H]^+$ ber. für $C_{45}H_{40}BrN_8O_{12}$: 963.1944, gef. 963.1949, $R_T$ = 7.93 min.

Compound 8

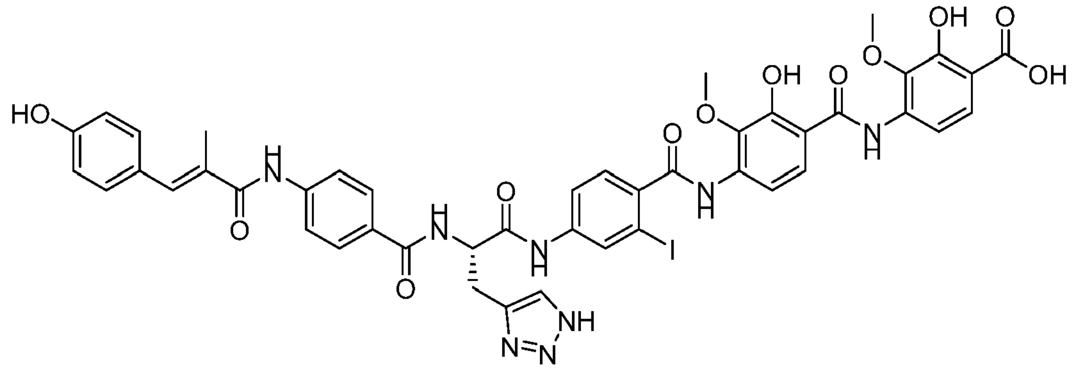

**$^1$H NMR** (700 MHz, DMSO-$d_6$) δ 11.58 (s, 1H), 11.52 (s, 1H), 11.19 (s, 1H), 10.42 (s, 1H), 10.08 (s, 1H), 9.96 (s, 1H), 9.76 (s, 1H), 8.68 (d, *J* = 7.6 Hz, 1H), 8.28 (d, *J* = 2.0 Hz, 1H), 8.06 (d, *J* = 8.9 Hz, 1H), 7.88 - 7.84 (m, 2H), 7.81 (dt, *J* = 9.2, 2.0 Hz, 3H), 7.76 - 7.71 (m, 1H), 7.67 (dd, *J* = 8.4, 2.0 Hz, 1H), 7.59 (d, *J* = 8.8 Hz, 1H), 7.46 (d, *J* = 8.3 Hz, 1H), 7.35 (d, *J* = 8.7 Hz, 2H), 7.26 (d, *J* = 1.6 Hz, 1H), 6.84 (d, *J* = 8.6 Hz, 2H), 4.86 (q, *J* = 7.5 Hz, 1H), 3.91 (s, 3H), 3.78 (s, 3H), 3.29 (dd, *J* = 14.8, 5.8 Hz, 1H), 3.22 (dd, *J* = 14.7, 9.0 Hz, 1H), 2.11 (d, *J*

= 1.4 Hz, 3H).

**HRMS (ESI-ORBITRAP):** *m/z* [M+H]$^+$ ber. für $C_{45}H_{40}IN_8O_{12}$: 1011.1805, gef. 1011.1799, $R_T$ = 8.39 min.

Test for biological activity

*Strains:*

*E. coli* BW25113, S. *typhimurium* TA100; *Bacillus subtilis* DSM10; and *M. phlei* DSM 750 and human serum

*Biological testing:*

The tests were performed using the micro dilution method.

Microdilution assay:

The determination of MIC values was performed according to the ninth edition of the Approved Standard M07-A9 (CLSI. Methods for Dilution Antimicrobial Susceptibility Tests for Bacteria That Grow Aerobically; Approved Standard-Ninth Edition. CLSI document M07-A9. Wayne, PA: Clinical and Laboratory Standards Institute; 2012.)

The test was carried out for different bacterial strains (E.coli DSM 1116, *E. coli* BW25113 [gram negative], B. subtilis DSM 10 [gram positive], M. luteus DSM 1790 [gram positive], S. typhimurium TA100 [gram negative]) and *M. phlei* DSM 750 . 20 μL of cryo stock of each strain were inoculated in 20 mL of LB media (Lysogeny broth: 10 g/L peptone, 5 g/L yeast extract, 5 g/L NaCl) followed by incubation over night at 37°C, 200 rpm. The test inoculum was adjusted by the 0.5 McFarland Standard (OD625 from 0.08 to 0.1). Within 15 min of preparation, the adjusted inoculum suspension was diluted in MHBII media (BBL TM Mueller-Hinton Broth II, Becton, Dickinson and Company, New Jersey/USA) so that each well contained approximately 5 x 105 CFU/mL in a final volume of 100 μL. 95 μL of the inoculum were applied per well and 5 μL of the (diluted) antibiotic substance were added.

Previously the dry antibiotic compounds were dissolved in DMSO (100%) with a concentration of 2560 μg/mL and the resulting stock solutions were further diluted in DMSO (100%). 5 μL of each antibiotic dilution were applied to the microdilution tray to reach final concentrations of 64 μg/mL to 0.008 μg/ml. One row of each well plate was left as a growth control without antibiotic substances and another row of the microdilution tray was used as sterility control (only MHB II-media). The antimicrobial effect of the solvent (DMSO) was tested by adding 5 μL DMSO to several wells without antibiotics. Purity check and cell titer control were performed according to International Standard M07-A9 on Mueller-Hinton II Agar (Mueller Hinton II Broth, 15 g/L agar-agar). Both microdilution trays and agar plates were incubated at 37°C for 20 h and subsequently analyzed visually.

The results are summarized in table 1. The potency of an antibiotic is determined by the minimum inhibitory concentration (MIC). Contrary to intuition, a particularly low value is equated with a high potency.

The following compound (0) is used as reference and lead structure and is of the following structure:

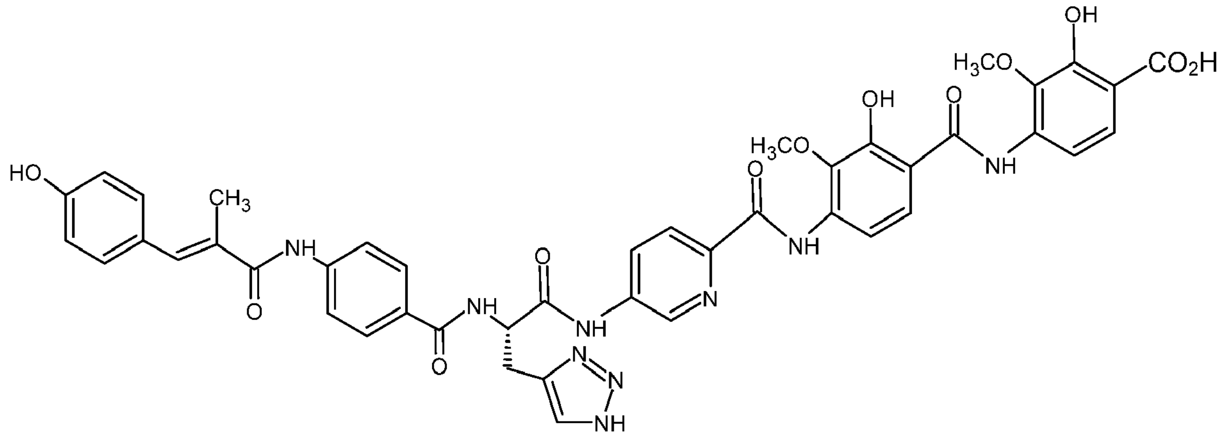

| Compound | | 0 | 1 | 2 | 3 | 5 |
|---|---|---|---|---|---|---|
| ***Gram-negativ*** | *E. Coli BW25113* | 0.015 | 0.016 | 0.015 | 0.015 | 0.125 |
| | *S.* Typhimirium | 0.015 | 0.015 | 0.015 | 0.015 | 0.031 |
| | **50% human serum *E. Coli.*** | **0.5** | **1** | **0.5** | **0.5** | **9** |
| ***Gram-positiv*** | *B. Subtilis* | 0.031 | 0.125 | 0.125 | 0.063 | 4.0 |
| | *M. phlei* | 0.125 | 1 | 1 | 0.5 | 8.0 |

*[≥8.0= 9; ≤ 0.016 = 0.015]*

Table 1: Antibacterial activity of compounds according to the invention against selected strains

Among the D-variations in Table 1, compounds 2 (difluoro) and 3 (chloro) should be highlighted. Due to their electronegative substituents and the H-bonds formed as a result, they are about as active as the lead structure (0). In contrast, however, they have a higher steric hindrance in the space of the D-E amide bond, this could prevent the attack of the serine endopeptidase, especially from the larger Cl atom to one.

**Claims**

1. Compound **characterized by** the general formula (1)

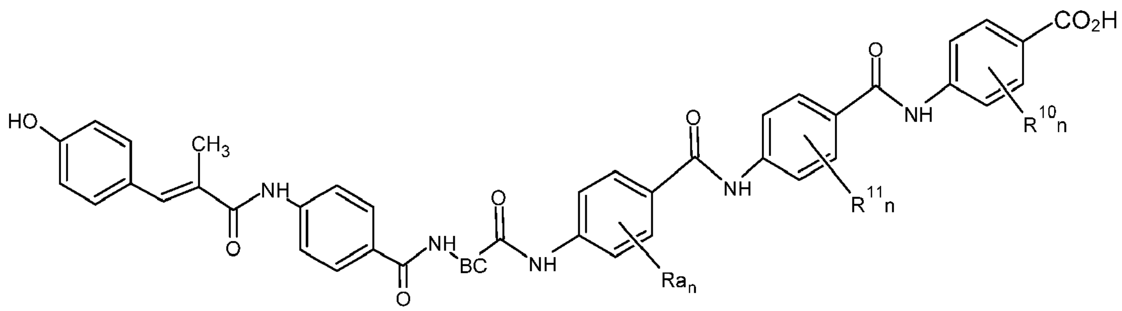

a) with BC being

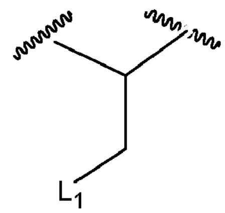

with $L_1$ being a substituted or unsubstituted C5-C6 aromatic heterocycle,
b) with n of $R^{10}_n$ and n of $R^{11}_n$ being independently from each other 0, 1, 2, 3 or 4, in particular n of $R^{10}_n$ and n of $R^{11}_n$ being 0, 1, 2 or 3, more particular n of $R^{10}_n$ and n of $R^{11}_n$ being 1 and 2;
with each $R^{10}$ and $R^{11}$ being selected independently from any other $R^{10}$ and $R^{11}$ from -OH, -F, -Cl, -Br, -I, -CCH, -CN, -$OC_1$-$C_6$ alkyl, in particular from -OH, -F, -$OCH_3$, - $OC_2H_5$, -$OiC_3H_7$, -$OnC_3H_7$,-$OCF_3$,; and
c) with n of $R^a_n$ being 0, 1, 2, 3 or 4, in particular 0, 1, 2 or 3, more particular being 1 and 2,
with $R_a$ being a substituent selected from halogen, in particular-Cl, - F, - Br, -I, - $NO_2$, -$NH_2$, -$CO_2H$, -CN, - $CF_3$,

**2.** Compound according to claim 1, **characterized in that** Ra being an electron withdrawing moiety selected from -Cl, - F, - Br, -I, - $CF_3$.

**3.** Compound according to one of the preceding claims, **characterized in that** $L_1$ is a five membered aromatic N-heterocycle, in particular a substituted or unsubstituted imidazole or triazole, most preferably a triazole.

**4.** Compound according to one of the preceding claims, **characterized in that** n of $R^{10}_n$ and n of $R^{11}_n$ being 0, 1, 2, 3 or 4, in particular n of $R^{10}_n$ and n of $R^{11}_n$ being 0, 1, 2 or 3, and with each $R^{10}$ and with each $R^{11}$ independently from any other $R^{10}$ being selected from -OH, -$OCH_3$, -$OC_2H_5$ or -OiPr, particularly with one $R^{10}$ or $R^{11}$ being -OH and the other $R^{10}$ or $R^{11}$ being -$OCH_3$, -$OC_2H_5$ or -OiPr respectively.

**5.** Compound according to one of the preceding claims, **characterized by** the general formulae (2)

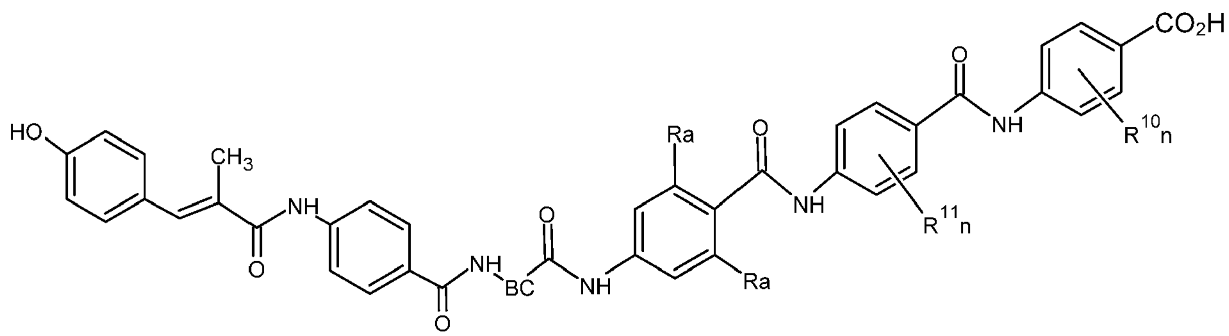

with $R^a$ being a substituent selected from halogen, in particular -Cl, - F, - Br, -I, -$NO_2$, -$NH_2$, -$CO_2H$, -CN, - $CF_3$, with BC, $R^{10}$n and $R^{11}$n as in one of the preceding claims.

**6.** Compound according to one of the preceding claims, **characterized by** the general formulae (3)

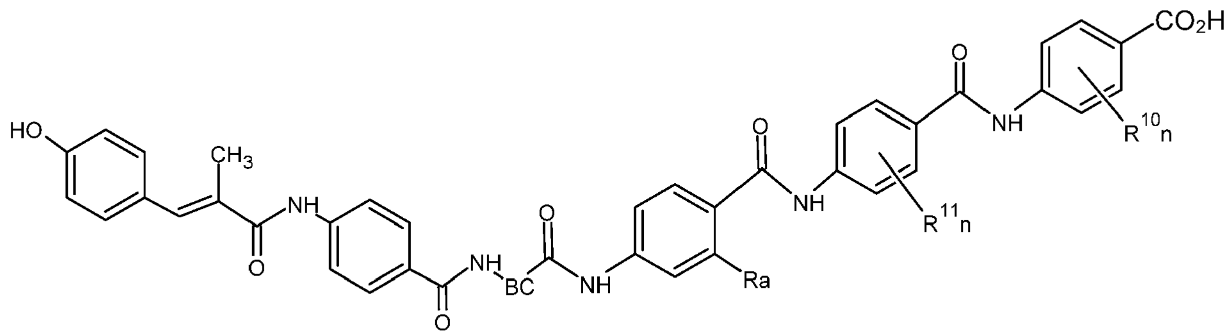

with $R^a$ being a substituent selected from halogen, in particular-Cl, - F, - Br, -I, $-NO_2$, $-NH_2$, $-CO_2H$, -CN, - $CF_3$, with BC, $R^{10}$n and $R^{11}$n as in one of the preceding claims.

**7.** Compound according to one of the preceding claims, **characterized by** the general formulae (4)

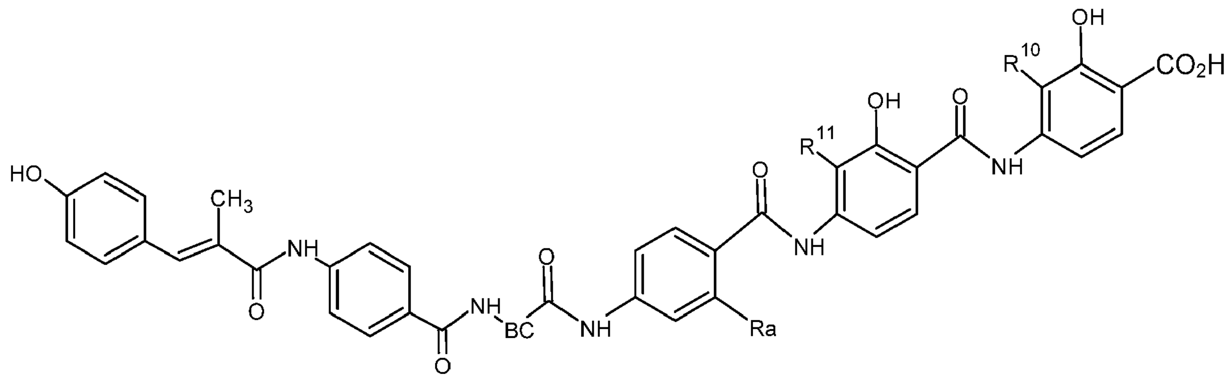

with $R^a$ being a substituent selected from halogen, in particular-Cl, - F, - Br, -I, $-NO_2$, $-NH_2$, $-CO_2H$, -CN, - $CF_3$, with BC, $R^{10}$ and $R^{11}$ as in one of the preceding claims.

**8.** Compound according to one of the preceding claims, **characterized by** the general formulae (5)

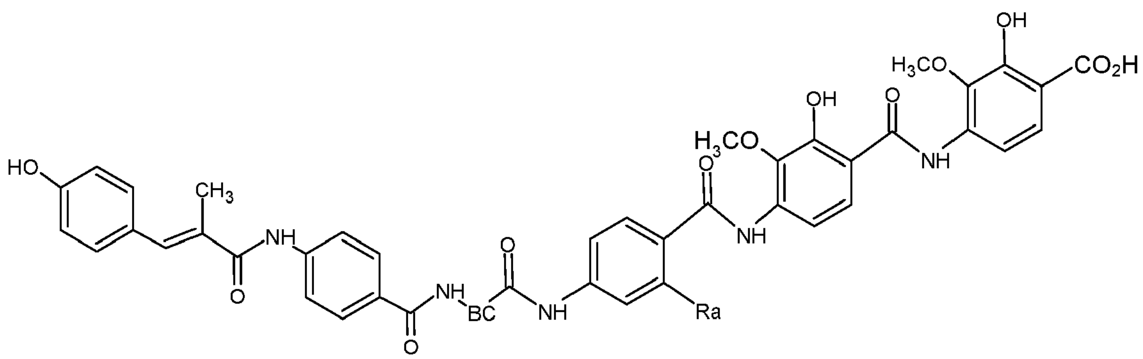

with $R^a$ being a substituent selected from halogen, in particular-Cl, - F, - Br, -I, $-NO_2$, $-NH_2$, $-CO_2H$, -CN, - $CF_3$, with BC as in one of the preceding claims.

**9.** Compound according to one of the preceding claims for use in a method of treatment of diseases, in particular for use in a method of treatment of bacterial infections by gram-negative or gram-positive bacterial strains.

**10.** Compound for use in a method according to claim 9, wherein the bacterial infection is an infection by one of the genus Acinetobacter, Bordatella, Borellia, Brucella, Camphylobacter, Chlamydia, Chlamydophila, Enterobacter, Escherichia, Francisella, Haemophilus, Helicobacter, Klebisella, Legionella, Leptospira, Morganella Moraxella, Neisseria, Proteus, Pseudomonas, Rickettsia, Shigella, Salmonella, Stenotrophomonas, Treponema or Yersinia, Bacillus, Chlostridium, Corynebacterium, Enterococcus, Listeria, Micrococcus, Staphylococcus or Streptococcus Mycobacterium, Mycoplasmataceae, in particular Escherichia, Bacillus, Salmonella, Micrococcus, Mycobacterium.

Europäisches Patentamt
European Patent Office
Office européen des brevets

# EUROPEAN SEARCH REPORT

Application Number
EP 22 17 6144

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/015794 A1 (UNIV BERLIN TECH [DE]) 24 January 2019 (2019-01-24) * claims 1-14; compounds 20, 23, 24, 25 * ----- | 1-10 | INV. C07D249/04 A61P31/04 |
| X | KLEEBAUER LEONARDO ET AL: "Overcoming AlbD Protease Resistance and Improving Potency: Synthesis and Bioactivity of Antibacterial Albicidin Analogues with Amide Bond Isosteres", ORGANIC LETTERS, vol. 23, no. 18, 16 August 2021 (2021-08-16), pages 7023-7027, XP055979429, US ISSN: 1523-7060, DOI: 10.1021/acs.orglett.1c02312 Retrieved from the Internet: URL:https://pubs.acs.org/doi/pdf/10.1021/acs.orglett.1c02312> | 1,3,4,9, 10 | |
| A | * compound 2 * ----- | 2,5-8 | TECHNICAL FIELDS SEARCHED (IPC) |
| X | IRAJ BEHROZ ET AL: "Extensive Structure-Activity Relationship Study of Albicidin's C-Terminal Dipeptidic p-Aminobenzoic Acid Moiety", CHEMISTRY - A EUROPEAN JOURNAL, JOHN WILEY & SONS, INC, DE, vol. 25, no. 72, 11 December 2019 (2019-12-11), pages 16538-16543, XP071850430, ISSN: 0947-6539, DOI: 10.1002/CHEM.201904752 * table 1; compounds 3-20 * ----- | 1,3,4,9, 10 | C07D A61P |

The present search report has been drawn up for all claims

1

| Place of search | Date of completion of the search | Examiner |
|---|---|---|
| Munich | 9 November 2022 | Sáez Díaz, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 17 6144

This annex lists the patent family members relating to the patent documents cited in the above-mentioned European search report. The members are as contained in the European Patent Office EDP file on
The European Patent Office is in no way liable for these particulars which are merely given for the purpose of information.

09-11-2022

| Patent document cited in search report | Publication date | Patent family member(s) | Publication date |
| --- | --- | --- | --- |
| WO 2019015794 A1 | 24-01-2019 | CA 3070240 A1 | 24-01-2019 |
| | | CN 110997641 A | 10-04-2020 |
| | | EP 3655394 A1 | 27-05-2020 |
| | | JP 2020527149 A | 03-09-2020 |
| | | US 2020165214 A1 | 28-05-2020 |
| | | WO 2019015794 A1 | 24-01-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Remington's Pharmaceutical Sciences. 1985, 1418